# EUROPEAN PATENT APPLICATION

(11) **EP 4 292 515 A1**
(43) Date of publication of application: **20.12.2023**
(21) Application number: 22179392.0
(22) Date of filing: 16.06.2022
(51) Int. Cl.: A61B 5/00, A61B 5/103, A61B 5/145

(54) **NON-CONTACT SUBJECT MONITORING**

(71) Applicant: Koninklijke Philips N.V., 5656 AG Eindhoven (NL)
(72) Inventor: VERHAGEN, Rieko, Eindhoven (NL); PALERO, Jonathan Alambra, Eindhoven (NL); SCHUIJERS, Erik Gosuinus Petrus, Eindhoven (NL); DAMODARAN, Mathivanan, Eindhoven (NL); LONG, Xi, Eindhoven (NL); ASSELMAN, Michel Jozef Agnes, Eindhoven (NL); MEFTAH, Mohammed, Eindhoven (NL); JOSHI, Rohan, Eindhoven (NL)
(74) Representative: Philips Intellectual Property & Standards

(57) **Abstract**

Provided is a computer implemented method (100) suitable for monitoring at least one skin region of a subject capable of moving relative to a source of ambient light. A sequence of video frames is received (102) of the subject. The sequence of video frames was captured while a light emitter provided a varying illumination, e.g. a pulsed illumination, to the subject. The sequence of video frames is then used to generate (106, 110) a first and second ambient light corrected image which are each then segmented (108, 112) to identify at least one skin region. Further provided is a computer program product for implementing the method, and a system for monitoring the at least one skin region.

## Description

### FIELD OF THE INVENTION

The present invention relates to a method suitable for monitoring the skin of a subject, such as a baby, and in particular for correction of ambient light when monitoring the skin of a subject that is capable of moving relative to a source of ambient light.

### BACKGROUND OF THE INVENTION

Visual inspection methods for monitoring the skin of a subject are known. Such visual inspection methods, for example routine skin colour analysis, are typically performed by a trained nurse who assesses the yellowness and the redness of the skin in order to identify if a subject is healthy, feeling comfortable and not in any kind of distress. This colour assessment suffers, however, from being subjective, performed on an irregular basis and may, in itself, cause distress to the subject, particularly a baby. The visual perception of colour is highly influenced by the colour rendering of the available light emitters and therefore proper colour assessment often has to be done under (near) daylight conditions. This can be difficult in certain settings, such as a newborn intensive care unit (NICU) and/or may necessitate the use of special light emitters with high colour rendering index.

A patient being monitored, either at home or in a hospital setting, such as the NICU may require regular and quantitative assessment in order to identify clear trend lines of a particular parameter and to perform a proper diagnosis. For example, for the quantitative measurement of bilirubin, a yellow pigment resulting from the breakdown of red blood cells, either blood testing or an assessment using a spectrometer based system such as the so-called BiliCheck is required. As another example, for the measurement of blood oxygenation, Photoplethysmography (PPG) contact probes are most often used that are fixed to the skin of the subject, for example a baby, by means of tape to secure their position, leading to skin irritation and blisters. These conventional intrusive measurement methods can cause significant discomfort to the subject and thus should be minimized, particularly when the subject is an infant or baby. There is thus a need for non-contact solutions that unobtrusively monitor the health state of babies, especially in the NICU.

Furthermore, it is desirable that the method for performing such an assessment can be done remotely such that the subject is not required to regularly attend a physician's office or a hospital. For remote continuous monitoring of skin colour under varying lighting conditions, the effect of the environment on the measurement result should be minimal in order for the measurement results to be clinically relevant. Whilst there are known inspection methods for monitoring the health of a subject that are non-invasive in which an image captured of the subject is processed to correct for the effects of ambient light, it may be challenging to keep the subject from moving for long enough a period of time for the method to take place. Because the monitoring of babies is so important, both in clinics and at home, non-contact remote monitoring may offer the best means to monitor babies since it is non-obtrusive. One of the challenges in non-contact baby monitoring is the presence of a number of sources of errors or artefacts during remote data acquisition. These mainly include motion of babies and changes in ambient light.

### SUMMARY OF THE INVENTION

The invention is defined by the claims.

According to examples in accordance with an aspect of the invention, there is provided a computer implemented method suitable for monitoring at least one skin region of a subject capable of moving relative to a source of ambient light, the method comprising: receiving a sequence of video frames of the subject captured while a light emitter is controlled to provide a varying illumination to the subject; generating, using a varying exposure between video frames of a first part of the sequence of video frames caused by said varying illumination during the capture of said first part, a first ambient light-corrected image; segmenting the first ambient light-corrected image to generate a first segmented image in which the at least one skin region is identified; generating, using a varying exposure between video frames of a second part of the sequence of video frames caused by said varying illumination during the capture of said second part, a second ambient light-corrected image, the second part being temporally distinct from the first part; segmenting the second ambient light-corrected image to generate a second segmented image in which said at least one skin region is identified.

This method may enable assessment of a skin region of a subject capable of moving relative to a source, in particular a stationary source, of ambient light. By correcting a first part of the sequence for ambient light and generating a first segmented image, correcting a second part of the sequence for ambient light and generating a second segmented image, the same skin region of the subject can be assessed at different points in time within the same sequence of video frames. In this way, allowance can be made for movement of the subject relative to the source of ambient light, such that reliable data relevant to the skin region can be obtained in spite of the subject's movement.

It is noted that the method can be repeated, for example by performing the method again on a further sequence of video frames captured after the sequence of video frames.

Alternatively or additionally, the method may comprise generating third, fourth, fifth, and so on, segmented image(s) using temporally distinct third, fourth, fifth, and so on part(s) of the sequence.

The term "varying illumination" may refer to illumination which is controlled to oscillate between providing high exposure to the subject and low exposure to the subject. The low exposure to the subject may mean that, in at least some embodiments, there is no illumination provided by the light emitter.

The variation of illumination provided is preferably achieved by pulsed illumination.

The pulsed illumination may also be regarded as being a modulated illumination.

The term "pulsed illumination" may refer to illumination which is controlled to be on during each pulse and off before and after each pulse.

In at least some embodiments, the pulsed illumination is periodic.

Any suitable waveform for the pulsed illumination can be contemplated, for example square wave or sinusoidal. Preferably, the light emitter is controlled to be off, in other words not providing, or providing only negligible, illumination, at a point (or points) during each period of the waveform.

In some embodiments, the same period of the pulsed illumination is applied when capturing the first part and the second part of the sequence of video frames.

The term "ambient light correction" can be regarded, in at least some embodiments, as an ambient light removal wherein the effect of the ambient light on the subject is removed so that assessment of the at least one skin region, for example a colour-related assessment of the at least one skin region, can be made.

The term "temporally distinct" may mean in this context that at least some of the frames of the second part are captured during a time period different, for example subsequent, to a time period during which the frames of the first part are captured.

In some embodiments, each of the frames of the first part is captured during a time period that is distinct from a time period during which each of the frames of the second part is captured. It is also conceivable that, in alternative embodiments, the first part and the second part share at least one common video frame for generating the first and second ambient light-corrected images.

It is noted that the terms "ambient light-corrected image" and "segmented image" are intended to refer to image data and hence neither of these terms should be understood as being limited to images displayable via a display device.

Hence, in some embodiments described herein the method comprises extracting at least one skin parameter from one or more of the first and second segmented images, but without displaying any of the first and second light-corrected images and the first and second segmented images.

In alternative embodiments, the method can further comprise displaying the first and second segmented images.

In such embodiments, a user, such as a clinician, carer and/or the subject, can make a visual comparison between the displayed first and second segmented images.

In some embodiments, the method comprises displaying the first and second segmented images and extracting at least one skin parameter from one or more of the first and second segmented images.

In some embodiments, the varying illumination may be a pulsed illumination; optionally wherein a pulse frequency of the pulsed illumination is at a frequency of at least 70Hz.

In other words, the varying illumination previously used while capturing the sequence of video frames may be a pulsed illumination, optionally wherein a pulse frequency of the pulsed illumination is at a frequency of at least 70Hz.

Light provided at a frequency of at least 70Hz may help in minimizing discomfort, or possible harm, to the subject while also assisting to provide ample illumination during the capture of the sequence of video frames to facilitate the ambient light correction.

In some embodiments, the method comprises controlling the light emitter to provide the varying illumination to the subject.

In such embodiments, the method may comprise controlling the light emitter to vary light at the frequency of at least 70Hz.

A frame rate of the sequence of video frames may be at least 24 frames per second.

The term "frame rate" may refer to a presentation rate of the, e.g. stored, sequence of video frames used for the ambient light correction and segmentation steps.

A frame/presentation rate of at least 24 frames per second, preferably at least 60 frames per second, can assist to ensure effective tracking of the moving subject's skin region(s).

In some embodiments, the sequence of video frames may be captured at a capture rate of at least 24 frames per second, preferably at least 60 frames per second. This may allow a sufficient number of frames to be captured for the sequence of video frames such that there is ample data available for the ambient light correction.

In other words, the capture rate previously used to capture the sequence of video frames may be at least 24 frames per second.

In some embodiments, the frame rate is equal to the capture rate.

In some embodiments, the method comprises controlling an imaging unit to capture the sequence of video frames.

In such embodiments, the method may comprise controlling the imaging unit to capture the video frames at the capture rate of at least 24 frames per second, and preferably at the capture rate of at least 60 frames per second.

In some embodiments, the video frames are rolling shutter camera-captured video frames.

The above-mentioned imaging unit may thus comprise, or be defined by, a rolling shutter camera.

In some embodiments, a timing of the varying illumination relative to a video frame capture timing is such that each of the video frames comprises more exposed areas caused by the varying illumination and less exposed areas whose exposure is due only to the source of ambient light, the more exposed and the less exposed areas being positioned differently between the video frames of the first part, and the more exposed and the less exposed areas being positioned differently between the video frames of the second part, wherein the first ambient light-corrected image is generated based on a comparison between the more exposed areas and the less exposed areas of the first part of the sequence of video frames, and wherein the second ambient light-corrected image is generated based on a comparison between the more exposed areas and the less exposed areas of the second part of the sequence of video frames.

The comparison between the more and less exposed areas of the respective part of the sequence of video frames can be implemented in any suitable manner in order to generate the respective ambient light-corrected image. For example, a pixel intensity corresponding to one of the areas in an image region can be subtracted from a pixel intensity corresponding to a more exposed area in the same image region. Thus, the subtraction results in a pixel intensity due to the illumination of the emitter rather than the source of ambient light.

More generally, the method may comprise linearizing each of the video frames prior to generating the first and second ambient light-corrected images.

This linearizing may include, or be defined by, applying a gamma correction to each of the video frames.

The frequency of the varying illumination may be inharmonic/not harmonic to the frame rate at which the video frames are captured.

Harmonics are periodic signals that have frequencies defined as a multiple of a fundamental frequency. Hence the term "inharmonic" in this context means that the frequency of the illumination provided by the light emitter is not a multiple integral of the frame rate at which the video frames are captured.

This inharmonic relationship between the frequency and the frame rate may help in achieving the desired more exposed areas and less exposed areas being positioned differently between the video frames of the first part and between the video frames of the second part such that they are suitable for the ambient light correction process.

In some embodiments, the method can further comprise: extracting, from one or more of the first and second segmented images, at least one skin parameter; and outputting the at least one skin parameter.

The above-described ambient light correction using the varying illumination and segmentation of the thus ambient light-corrected image may mean that the skin parameter(s) can be reliably extracted from the first and/or second segmented images.

When the skin parameter(s) is or are extracted from both of the first and second segmented images, the ambient light correction may assist in terms of enabling, in some embodiments, a valid comparison to be made between the skin parameter(s) extracted from the first segmented image to the corresponding skin parameter(s) extracted from the second segmented image. Thus, the skin parameter(s) can be tracked over time within the same sequence of video frames.

The skin parameter may be a measure of one or more of: skin colour, skin texture, skin shape, and skin optical reflectance.

Particular mention is made of the skin parameter being a measure of skin colour because extraction of this measure is greatly facilitated by the above-described ambient light correction and segmentation process. Such a measure of skin colour also finds particular utility in, for instance, assessing a bilirubin level in the subject, for example a neonate.

Thus, in some embodiments, the skin parameter includes, or is, a measure of the subject's bilirubin level.

Since the method of the present disclosure enables monitoring of the same skin region of the subject at different points in time within the same sequence of video frames, the measure of skin colour of the subject, for example neonate, can be closely monitored in spite of the subject's movement relative to the source of ambient light.

In some embodiments, the first and second segmented images each comprise a first skin region and a second skin region, the method comprising: comparing the first skin region identified from the first segmented image to the first skin region identified from the second segmented image to determine a first image consistency parameter; comparing the second skin region identified from the first segmented image to the second skin region identified from the second segmented image to determine a second image consistency parameter; and comparing the first and second image consistency parameters.

This comparison of image consistency parameters may assist in identifying which of the first and second skin regions is more appropriate for use in subsequent analysis, for example which of the first and second skin regions should be used for extraction of the one or more skin parameter(s).

The method may further comprise selecting the first skin region or the second skin region based on the comparison of the first and second image consistency parameters, with the outputted skin parameter being relevant to the selected skin region.

By selecting the skin region which is more consistent between the first and second segmented images, for example due to having a higher image consistency parameter, the skin parameter may be more reliably extracted, particularly in embodiments in which the method comprises extracting the skin parameter relevant to the selected skin region from the first segmented image and the second segmented image.

The method may further comprise determining, using the sequence of video frames, an image quality metric, wherein the extracting or the outputting of said skin parameter is implemented responsive to the image quality metric meeting or exceeding a predetermined threshold.

In this way, the skin parameter may only be extracted or outputted if the image quality metric meets or exceeds the predetermined threshold. This may assist to avoid a scenario in which an unreliable skin parameter is provided due to poor image quality.

The method may also comprise issuing, responsive to the image quality metric failing to meet said predetermined threshold, a report indicating unreliable image data; or outputting, responsive to the image quality metric meeting/exceeding the predetermined threshold, said skin parameter relevant to the at least one skin region.

Issuing such a report based on the image quality metric may allow for further steps to be taken which may help in obtaining further images that meet the predetermined threshold by, for example, re-positioning the subject and repeating the capture of the sequence of video frames.

In some embodiments, the method may include: obtaining RGB values of the first segmented image (204A) and/or the second segmented image (204B); and converting the RGB values to CIE L^{∗}a^{∗}b^{∗} values.

Such conversion to CIE L^{∗}a^{∗}b^{∗} values may be particularly useful when the skin parameter(s) includes or is a measure of skin colour. CIE L^{∗}a^{∗}b^{∗} values may facilitate, in particular, assessment of a bilirubin level in the subject, for example a neonate.

The method may include obtaining RGB values of the first segmented image 204A and/or the second segmented image 204B; converting the RGB values into XYZ tri-stimulus values; and converting the XYZ tri-stimulus values into CIE L^{∗}a^{∗}b^{∗} values.

According to another aspect there is provided a computer program product comprising computer program code which, when executed on a computing device having a processing system, causes the processing system to perform all of the steps of the method.

According to yet another aspect there is provided a system for monitoring at least one skin region of a subject capable of moving relative to a source of ambient light, the system comprising: a light emitter; an image capture unit; and a processing system configured to: receive a sequence of video frames of the subject captured by the image capture unit while the light emitter provides a varying illumination to the subject; generate, using a varying exposure between video frames of a first part of the sequence of video frames caused by said varying illumination during the capture of said first part, a first ambient light-corrected image; segment the first ambient light-corrected image to generate a first segmented image in which the at least one skin region is identified; generate, using a varying exposure between video frames of a second part of the sequence of video frames caused by said varying illumination during the capture of said second part, a second ambient light-corrected image, the second part being temporally distinct from the first part; and segment the second ambient light-corrected image to generate a second segmented image in which said at least one skin region is identified.

In some embodiments, the processing system may be further configured to control the image capture unit to capture said sequence of video frames; and/or to control the light emitter to provide said pulsed illumination to the subject.

One or more non-transitory computer readable media may be provided, which non-transitory computer readable media have a computer program stored thereon, with the computer program comprises computer program code which is configured, when the computer program is run on the one or more processors, to cause the one or more processors to implement the method according to any of the embodiments described herein.

More generally, embodiments described herein in relation to the method or computer program may be applicable to the system, and embodiments described herein in relation to the system may be applicable to the method or computer program.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 shows a flowchart of a method suitable for monitoring at least one skin region according to an example;
FIG. 2 schematically depicts a sequence of video frames and processing of first and second parts of the sequence of video frames in accordance with an example;
FIG. 3 shows a flowchart of a method suitable for monitoring at least one skin region according to another example;
FIG. 4 shows a flowchart of a method suitable for monitoring at least one skin region according to another example; and
FIG. 5 shows a block diagram of a system for monitoring at least one skin region according to an example.

### DETAILED DESCRIPTION OF THE EMBODIMENTS

The invention will be described with reference to the Figures.

It should be understood that the detailed description and specific examples, while indicating exemplary embodiments of the methods and systems, are intended for purposes of illustration only and are not intended to limit the scope of the invention. These and other features, aspects, and advantages of the methods and systems of the present invention will become better understood from the following description, appended claims, and accompanying drawings. It should be understood that the Figures are merely schematic and are not drawn to scale. It should also be understood that the same reference numerals are used throughout the Figures to indicate the same or similar features.

Provided is a computer implemented method suitable for monitoring at least one skin region of a subject capable of moving relative to a source of ambient light. A sequence of video frames is received of the subject. The sequence of video frames was captured while a light emitter provided a varying illumination, e.g. a pulsed illumination, to the subject. The sequence of video frames is then used to generate a first and second ambient light corrected image which are each then segmented to identify at least one skin region. Further provided is a computer program product for implementing the method, and a system for monitoring the at least one skin region.

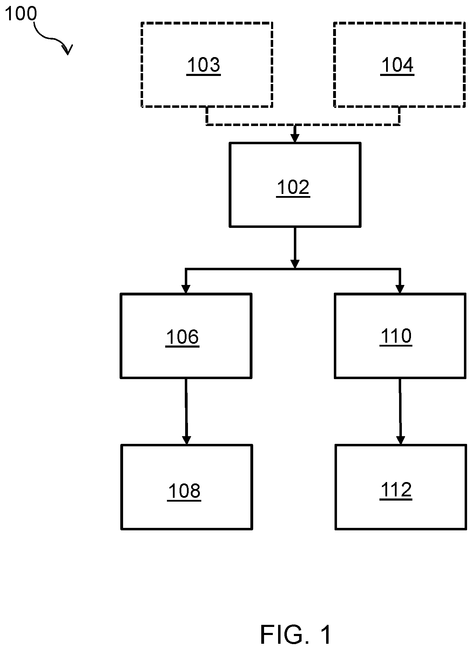

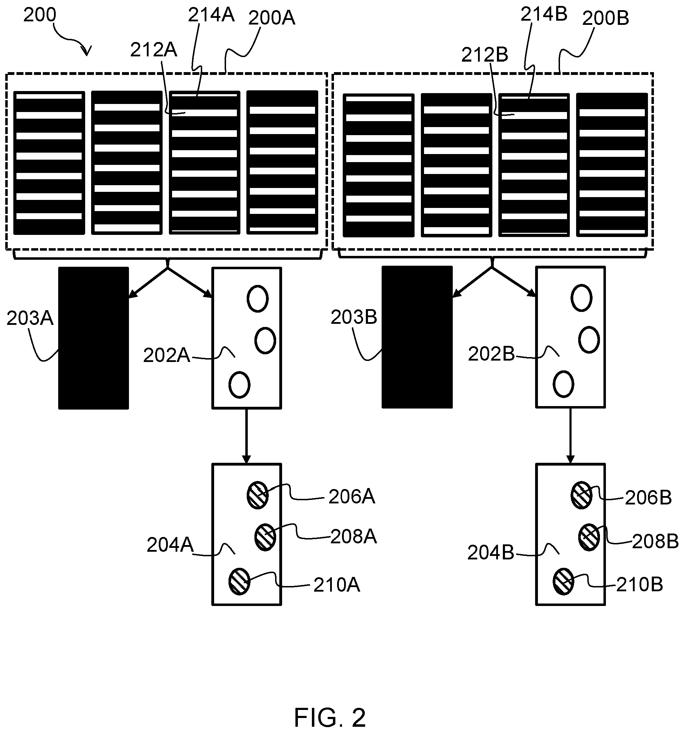

FIG.1 shows a flowchart of a computer implemented method 100 according to an embodiment, which can be used for performing ambient light correction suitable for monitoring at least one skin region of a subject capable of moving relative to a source of ambient light. Referring to FIGs. 1 and 2, the method 100 comprises receiving 102 a sequence of video frames 200, generating 106, 110 a first and second ambient light-corrected image, and segmenting 108, 112 the first and second ambient light-corrected images to identify the at least one skin region.

Referring to FIG. 2, the sequence of video frames 200 is for example multiple video frames or a video stream of a subject obtained chronologically by an image capture unit, such as a rolling shutter camera. The sequence of video frames 200 is of a subject such as a patient, for example an infant, whose skin is to be monitored. The received sequence of video frames 200 has been captured by the image capture unit which has been controlled 104 to capture said video frames 200 while a light emitter was controlled 103 to provide varying illumination to the subject.

The light emitter and the image capture unit may have been controlled to illuminate the subject in any suitable way such that the resulting video frames comprise varying areas of exposure 214A, 214B, 212A, 212B. In some embodiments, the light emitter may have been controlled to be on only while every other frame is being captured by the image capture unit. In this way, the sequence of video frames 200 alternates between a video frame in which the subject is illuminated by the light emitter and a video frame in which the subject is illuminated by ambient light only.

Alternatively, the light emitter and the image capture unit may have been controlled such that the exposure to the subject varies within each individual video frame. Referring again to FIG. 2, the varying illumination has caused the video frames 200 to comprise bright and dark bands in each video frame. The bright bands (the more exposed areas 214A, 214B) show the areas where the subject was illuminated by the light emitter during the capture of the video frame and the dark bands (less exposed areas 212A, 212B) show the areas where the subject was not, or minimally illuminated by the light emitter. The varying illumination from the light emitter may cause the video frames to comprise more exposed areas and less exposed areas in which the boundaries between the areas are blurred, i.e. less defined than a bright and dark band. In other words, the transition between the more exposed areas to the less exposed areas is gradual.

The varying illumination can refer to illumination which is controlled to oscillate between providing high exposure to the subject and low exposure to the subject. The high exposure can refer to a period in which the subject is illuminated by the light emitter. The low exposure can refer to a period in which the subject is illuminated by the ambient light only. In alternative embodiments, the low exposure can mean that there is minimal illumination provided by the light emitter to the subject, such as in the case where the light emitter is pulsed between a high and low point during each period of a light waveform.

The varying illumination or pulsed illumination may, in some embodiments, be regarded as being a modulated illumination. The term "modulated illumination" may refer to illumination which is controlled to vary in strength or intensity as it illuminates the subject. As such, the illumination can vary in intensity and frequency across each video frame and thus can cause the more exposed areas and less exposed areas to vary in, for example, intensity of the illumination on the subject and/or size of the more and less exposed areas.

When using pulsed illumination, the illumination can be controlled to be on during each pulse and off before and after each pulse. As such, this provides clear areas of low exposure to the subject and areas of high exposure to the subject through the sequence of video frames 200. In this way, the pulsed illumination is periodic and can be of any suitable waveform such as square wave or sinusoidal. It may be preferable that the light emitter is controlled to be off, in other words not providing, or providing only negligible, illumination, at a point (or points) during each period of the waveform such that there is a clear distinction between the more and less exposed areas in the sequence of video frames.

If the varying illumination does not reach a zero-level (controlled to be off), mathematically the driving waveform can be biased by a DC or constant signal. This DC bias can be considered as an ambient light contribution. As will be discussed further below, this is not ideal for ambient light correction as the generated ambient light corrected image will have a lower dynamic range compared to an ambient light corrected image that is generated using a varying illumination that goes to zero levels (i.e. is controlled to be off at some point during the capture of the video frames).

The method may include the controlling 103 of the light emitter to provide the varying illumination to the subject. In some embodiments, the varying illumination is a pulsed illumination; optionally wherein a pulse frequency of the pulsed illumination is at a frequency of at least 70Hz.

Alternatively or additionally, the method may include the controlling 104 the imaging unit to capture the video frames. In some embodiments the imaging unit may be controlled to capture the video frames at a capture rate of at least 24 frames per second, and preferably at a capture rate of at least 60 frames per second.

The sequence of video frames 200 is then processed to generate 106, 110 a first and second ambient light-corrected image 202A, 202B. FIG. 2 schematically depicts the sequence of video frames 200 and processing of first and second parts 200A, 200B of the sequence of video frames 200 in accordance with an embodiment.

In the embodiment shown in FIG. 2, the sequence of video frames 200 comprises eight video frames to be used in generating 108,110 the first and second ambient light-corrected images 202A, 202B. Any number of video frames of three or more in the sequence of video frames can be used. The sequence of video frames is sectioned into a first part 200A and a second part 200B, each comprising four different video frames, in other words the video frames of the first part 200A and the second part 200B are temporally distinct from one another. Alternatively, each of the frames of the first part is captured during a time period that is distinct from a time period during which each of the frames of the second part is captured. In further alternative embodiments, the first part and the second part share at least one common video frame for generating the first and second ambient light corrected images.

In the embodiment shown, each of the video frames comprises more exposed areas 212A, 212B and less exposed areas 214A, 214B. These more exposed areas 212A, 212B and less exposed areas 214A, 214B are a result of the timing of the varying illumination, or pulsed illumination, being relative to a video frame rate capture timing such that the subject is illuminated for at least portion of time less than the time in which the video frame is captured. In other words the frequency of the illumination is higher than the rate at which the camera, such as a rolling shutter camera, captures the sequence of video frames. In the sequence of video frames 200 shown in FIG.2, the subject is exposed to pulsed illumination with a frequency higher than that of the rate at which a rolling shutter camera captured the image, resulting in the more exposed areas 212A, 212B and less exposed areas 214A, 214B.

The same period of the pulsed illumination may be applied when capturing the first part 200A and second part 200B as in the illustrative example shown in FIG. 2. In alternative embodiments the period of the pulsed illumination can vary between the first part 200A and the second part 200B. However, in the video frames of the first part 200A and the second part 200B, each more exposed area 212A, 212B of one video frame has a corresponding less exposed area 214A, 214B in a corresponding video frame within the respective first or second part 200A, 200B. This allows for a more complete ambient light corrected image 202A, 202B to be generated 108, 110, as will be discussed further below.

The more exposed areas 212A, 212B and less exposed areas 214A, 214B are compared to each other to generate a first and second ambient light-corrected image 202A, 202B. This comparison between the more and less exposed areas of the respective part of the sequence of video frames can be implemented in any suitable manner in order to generate the respective ambient light-corrected image. In some embodiments, this is achieved by comparing a first composite image (not visible in FIG. 2) comprising the contributions of the light emitter to the subject (the more exposed areas 212A, 212B) and a second composite image 203A, 203B comprising the contributions of the ambient light only to the subject (the less exposed areas 214A, 214B) such that the effect of ambient light can be removed from the video frames. For example, a derived pixel intensity of the less exposed 214A, 214B areas can be subtracted from a derived pixel intensity of more exposed areas 212A, 212B such that the remaining pixel intensity contains the contribution of, at least mostly, the illumination provided by the light emitter. This remaining pixel intensity can be used to construct the ambient light-corrected images 202A, 202B. When the varying illumination is a pulsed illumination in which the light emitter is controlled to be on during each pulse and off before and after each pulse, a clear distinction between the more and less exposed areas is available, and thus deriving and subtracting the pixel intensity is can be performed.

Alternatively, when the illumination is controlled to oscillate between providing high exposure to the subject and low exposure to the subject, such as when the waveform is not a square wave and thus results in less distinct more and less exposed areas, further image processing may then be performed to decompose the set of images into the contribution of only the illumination provided by the light emitter and the contribution of only the ambient light.

In some embodiments the method may linearize each of the video frames prior to generating the first and second ambient light-corrected images 202A, 202B. This linearizing may include, or be defined by, application of a gamma correction to each of the video frames.

The ambient light corrected images 202A, 202B may be ambient light removed images 202A, 202B. The term "ambient light removed images" may refer to ambient light corrected images in which the effects of the ambient light are removed from the video frames.

In the embodiment shown in FIG. 2 the video frames 200 are a result of the frequency of the varying illumination being inharmonic/not harmonic to the frame rate at which the video frames are captured. Harmonics are periodic signals that have frequencies defined as a multiple of a fundamental frequency. Therefore, in the embodiments shown in FIG. 2, the frequency of the illumination provided by the light emitter is not a multiple integral of the frame rate at which the video frames are captured.

Thus, a first ambient light-corrected image 202A is generated 106 using the varying exposure between video frames of a first part 200A of the sequence of video frames 200 caused by the varying illumination during the capture of the first part. The second ambient light-corrected image 202B is generated 110 using a varying exposure between the video frames of a second part 200B of the sequence of video frames caused by the varying illumination during the capture of the second part 200B that is temporally distinct from the first part 200A.

The generated first ambient light corrected image 202A is then segmented 108 to generate a first segmented image 204A in which at least one skin region is identified 206A, 208A, 210A. Likewise, the generated second ambient light-corrected image 202B is then segmented 110 to generate a second segmented image 204A in which at least one skin region is identified 206B, 208B, 210B. In segmenting the first and second ambient light-corrected images 202A, 202B, at least one skin region 206A, 206B is identified across the first and second ambient light corrected images.

Segmenting 108, 112 to identify skin regions of interest can be carried out in any suitable way known in the art. For example, a sharp difference in a pixel characteristic, such as pixel intensity or colour, can be used to define the outline of a body part or skin region such as a mole or blemish. The ambient light-corrected image can then be segmented based on a detected difference in pixel characteristics to isolate a skin region of interest. Other regions of interest can also be segmented using this method, such as the general forehead, limb or other appendage.

In segmenting the first and second ambient light corrected images, the same skin region can be identified in both the first and second ambient light corrected images. This may be enabled by the ambient light correction being performed separately for each of the first ambient light corrected image and the second ambient light corrected image before segmenting. This allows both images to be compared to each other regardless of the change in ambient light conditions during the capture of the sequence of video frames 200 such that the difference in pixel characteristics which define the same skin region in each of the first and second ambient light corrected images are similar. For example if the subject were to move their head from one position to a different position between the first part of the sequence of video frames and the second part of the sequence of video frames, the effect of the ambient light on, for instance, the forehead would cause the same skin region to be differently illuminated. Thus by correcting for ambient light in each image before segmenting, the same skin region can be identified, compared and/or processed for monitoring.

Because the skin regions have been corrected for ambient light separately for the first segmented image and the second segmented image, a skin parameter can be extracted from the segmented images 204A, 204B. The ambient light correction assists in terms of enabling a valid comparison to be made between the skin parameter extracted from the first segmented image to the corresponding skin parameter extracted from the second segmented image. Thus, the skin parameter can be tracked overtime within the same sequence of video frames. Alternatively or additionally, the skin region can be tracked and displayed on a display unit for the user to visually inspect the skin region, after having been corrected for ambient light.

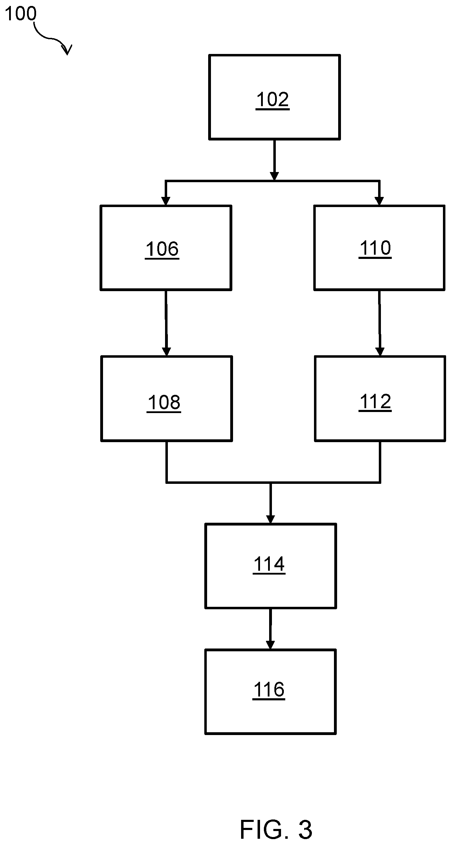

The skin parameter derived from the skin region can be extracted from the first and second segmented images and output to the user. FIG. 3 shows a flowchart of an embodiment which includes the further steps of extraction 114, from one or more of the first and second segmented images 204A, 204B, at least one skin parameter and outputting 116 the at least one skin parameter.

The outputted skin parameter can be a measure of one of more of skin color, skin texture, skin shape, and skin optical reflectance or any other useful measure applicable in monitoring or determining the health of a subject.The outputted skin parameter may also be a time-series of skin-based parameters used to obtain physiologically relevant time dependent parameter, for example time variation in skin redness to obtain a heart rate. This physiologically relevant parameter could also be reported/output to the user by a display or sound. The method may also include performing an algorithm to detect a subject's health state, for example normal or stressed state, based on the physiologically relevant parameter values in comparison to a lookup table, and could provide the user with an alarm, report, sound, to alert to a change in state.

In other embodiments, the method 100 can comprise processing the ambient light-corrected images to estimate the pose of the subject. This can be achieved in with any suitable means known in the art, such as using Artificial intelligence, Deep Learning, Convolutional Neural Networks (CNNs), and Fully Convolutional Networks (FCNs). In the case of FCNs, it would be desirable to include gated skip connections for controlling the data flow for each channel within the network for controlling the data flow. The method may then generate a skeleton image and/or a segmentation of body parts of the patient, otherwise known as semantic segmentation.

In some embodiments, an algorithm may be used for ambient light correction. Any suitable algorithm know in the art can be used, however some are known to be relatively sensitive to subject motion, causing intense white lines along the edges of significantly moving objects. Therefore, by default, the algorithm will establish the absolute colour based on intervals of minimal motion. Alternatively, and preferably, the method uses an algorithm that identifies which frames within a sequence have little motion artefacts and are therefore best suited for ambient light correction. Thus the method can select the video frames from the sequence of video frames to be used in the first part and second part for generating the first and second ambient light corrected images.

The method may also include obtaining RGB values of the first segmented image 204A and/or the second segmented image 204B and convert these RGB values to CIE L^{∗}a^{∗}b^{∗} values. The colours captured by most image capture units, such as a rolling shutter camera, are typically expressed in RGB numbers, the visual perception system of the human eye is much better represented by the CIE L^{∗}a^{∗}b^{∗} colour system. The method may first convert the RGB values to XYZ tri-stimulus values, the XYZ values being suitable for then converting to CIE L^{∗}a^{∗}b^{∗} values. Thus by converting the RGB values to CIE L^{∗}a^{∗}b^{∗} values, the skin regions can be analyzed or presented in a way more useful to the user, say a nurse practitioner.

Thus the method may include obtaining RGB values of the first segmented image 204A and/or the second segmented image 204B; converting the RGB values into XYZ tri-stimulus values; and converting the XYZ tri-stimulus values into CIE L^{∗}a^{∗}b^{∗} values.

In an exemplary embodiment, the ambient light removal from a sequence of video frames 200 captured using a light emitter of known spectrum, and a rolling shutter RGB camera enabled to capture a sequence at high framerate (60, 90 fps or higher) as described in the methods above. From the linear RGB values obtained in this way the tri-stimulus XYZ values and therewith the CIE-L^{∗}a^{∗}b^{∗} values can be determined. By compensating for ambient light changes, it is possible to compensate for artefacts that would otherwise be induced by for example clothing of staff and visitors passing by that could induce apparent colour changes of the illumination. Reporting a^{∗} and b^{∗} values as apparent redness and yellowness may provide much more relevant tracking of the patient's condition.

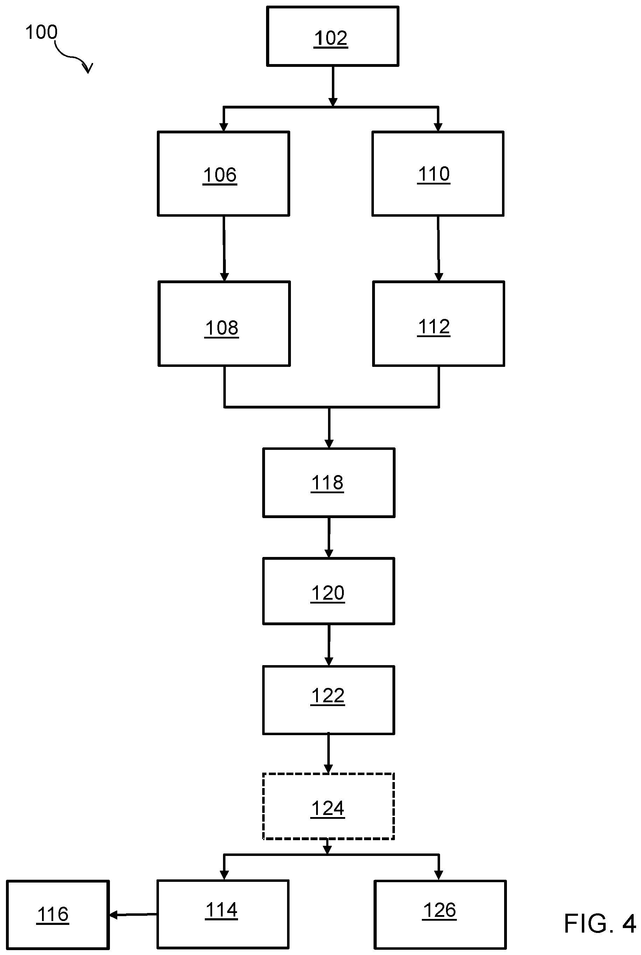

FIG. 4 shows a flowchart of method suitable for monitoring a skin region where the first and second segmented images 204A, 204B each comprise a first skin region 206A, 206B and a second skin region 208A, 208B. The first skin region 206A is identified from the first segmented image 204A and is compared 118 to the first skin region 206B identified from the second segmented image 204B to determine a first image consistency parameter. A second skin region 208A is then identified from the first segmented image and compared to a second skin region 208B identified from the second segmented image to determine a second image consistency parameter. The resulting first and second image consistency parameters are then compared to each other.

Thus, by comparing the image consistency parameters, the method identifies which of the first and second skin regions is more appropriate for use in subsequent analysis, such as outputting a skin parameter derived from the more appropriate of the first or second skin regions. The image consistency parameter can be based on pixel intensity, such that it identifies which of the first and second skin regions more closely match each other after ambient light correction.

FIG. 4 further depicts determining 124 using the sequence of video frames, an image quality metric. Determining 124 the image quality metric for each skin region is based on at least one parameter related to: signal-to-noise ratio of the subsequent video frames within the sequence of video frames; and motion between the subsequent video frames. The image quality metric may also partially come from probabilities generated by artificial intelligence algorithms.

The extracting 114 or the outputting 116 of the skin parameter is implemented responsive to the image quality metric meeting or exceeding a predetermined threshold. If the image quality metric fails to meet the predetermined threshold, a report indicating that the image data is unreliable is issued 126. If the image quality metric meets or exceeds the predetermined threshold, the skin parameter relevant to the at least one skin region is output 114.

Issuing such a report based on the image quality metric may allow for further steps to be taken which may help in obtaining further images that meet the predetermined threshold. For example, the report may recommend reposition of the subject and/or to receive a new sequence of video frames.

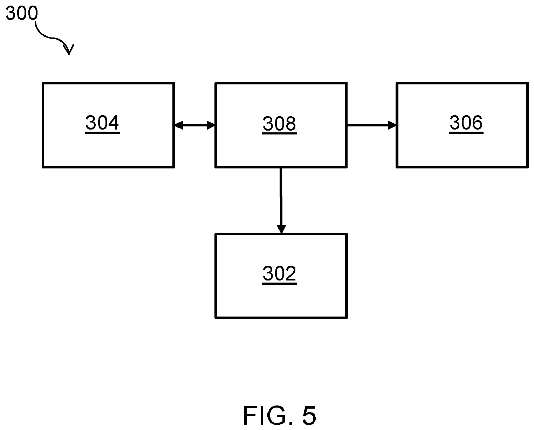

FIG. 5 depicts a system 300 for monitoring at least one skin region of a subject capable of moving relative to a source of ambient light. The system 300 comprises a light emitter 302, an image capture unit 304, and a processing system 308.

The system is arranged to receive the sequence of video frames of the subject captured by the image capture unit 304 while the light emitter 302 provides a varying illumination to the subject. The resulting varying exposure between the video frames of a first part of the sequence of video frames caused by the varying illumination during the capture of the first part is then used to generate a first ambient light-corrected image. The system then segments the first ambient light-corrected image to generate a first segmented image in which at least one skin region is identified.

The resulting varying exposure between the video frames of a second part of the sequence of video frames caused by the varying illumination during the capture of the second part is used to generate a second ambient light-corrected image. The system is arranged to then segment the second ambient light corrected image to generate a second segmented image in which the at least one skin region is identified.

In some embodiments the processing unit 308 is used to control the image capture unit 304 to capture the sequence of video frames 200. The processing unit 308 can be arranged to control the light emitter to provide the subject with the varying illumination. Thus the processing unit 308 can be arranged to control the image capture unit to capture the sequence of video frames of the subject while simultaneously controlling the light emitter to provide the varying illumination to the subject.

In some embodiments, the system 300 may further comprise at least one user interface 306. Alternatively of additionally, the at least one user interface may be external to, separate to or remote from the system 300. For example, at least one user interface may be part of another device. A user interface may be for use in providing a user of the system 300 with information resulting from the method described herein. Alternatively or additionally, a user interface may be configured to receive a user input. For example, a user interface may allow a user of the system 300 to manually enter instructions, data, or information. In these embodiments, the processing unit 308 may be configured to acquire the user input from one or more user interface(s).

A user interface may be any user interface that enables the rendering, output, or display of information to a user of the system 300. Alternatively or additionally, a user interface may be any user interface that enables a user of the system 300 to provide a user input, interact with and/or control the system 300. For example, the user interface may comprise one or more switches, one or more buttons, a keypad, a keyboard, a touch screen or an application (for example on a tablet or a smartphone), a display screen, a graphical user interface (GUI) or other visual rendering component, one or more speakers, one or more microphones or any other audio component, one or more light emitters, a component for providing tactile feedback, such as a vibration function), or any other user interface, or combination of user interfaces.

In some embodiments, the system 300 may comprise a memory. Alternatively or additionally, one or more memories may be external, separate to or remote from the system 300. For example, one or more memories may be part of another device. A memory can be configured to store program code that can be executed by the processing unit 308 to perform the method described herein. A memory can be used to store information, data, signals and measurements acquired or made by the processing unit 308 of the system 300. For example, one or more of the sequence of video frames 200, e.g. including the presentation rate of the sequence of video frames, the first ambient light corrected image 202A, the second ambient light corrected image 202B, the first segmented image 204A and the second segmented image 204B may be stored in the memory. The processing unit 308 may also be configured to control the memory to store the sequence of video frames 200, e.g. including the presentation rate of the sequence of video frames, the first ambient light corrected image 202A, the second ambient light corrected image 202B, the first segmented image 204A, and/or the second segmented image 204B.

In some embodiments, the system 300 may comprise a communication interface or circuitry for enabling the system 200 to communicate with any interfaces, memories and/or devices that are internal or external to the system 300. The communications interface may communicate with any interfaces, memories and/or devices wirelessly or via a wired connection. For example, the communications interface may communicate with one or more user interfaces wireless or via a wired connection. Similarly, the communications interface may communicate with the one or more memories wirelessly or via a wired connection.

The processing unit may comprise more than one computer processor unit (CPU), for example a first CPU to control the image capture unit and second CPU to control the light emitter.

The image capture unit 304 may comprise a CPU configured to send the captured sequence of video frames to the processing unit 308, arranged to receive the video frames for ambient light correction and segmentation. Thus the image capture unit 304 and the processing unit 308 are arranged to communicate with each other such that the processing unit 308 commands the image capture unit to capture a sequence of video frames and the image capture unit reciprocates by capturing and then sending the video frames to the processing unit.

The processing system 308 may include a CPU configured for: correcting the captured sequence of video frames by removal of ambient light; segmenting the captured sequence of video frames to relevant image segments for image analysis; computing skin parameter values; and converting the computed relevant skin parameter values to the subject's health state. The system may further include an interface unit for providing a subject's health state signal, for example "stop treatment", based on the converted subject's health state signal, for example average bilirubin.

The system may further comprise a treatment unit configured to receive the subject's health state signal from the interface unit. The system may also comprise a user interface unit configured to receive subject health state signal and provide a user relevant information, for example an alarm when a certain signal is above a threshold. The CPU further may be further configured to implement the step of detecting and measuring movement of a subject, such as a baby, resulting in a motion map, and hence image segmentation may be implemented with motion compensation.

The system may be arranged to capture a sequence of video frames of a subject at regular time intervals and calculate and store the ambient light corrected images. On request, the system may show on a display 306 these ambient light corrected images as a video on a colour display in the form of a time lapse. This would make the trend over time of colour change better visible to a human eye to distinguish improvement and change of the health conditions of said subject. Additionally the colour changes, if present, can be exaggerated by applying a multiplication factor >1 on the ^{∗}a and ^{∗}b values of the colours displayed.

In a further exemplary embodiment of the present invention is a camera-based baby monitoring system for home-use comprising an imaging device and a display device. The imaging device is in wireless communication, for example via Wifi, to the display device for bi-directional video, audio and data communication. The imaging device comprises a camera unit 304, modulated infrared LED (940 nm) unit 302, control unit 308, and computing unit. The camera unit is configured for use in the day and night time, thus with good spectral sensitivity in the 940 nm region. The control unit controls the camera unit and the modulated LED unit. The computing unit 308 computes the ambient-light corrected frames, and streams the video to the display device 306. The display device comprises a second computing unit for processing the video for segmentation into body image parts, and computing for the reliability score-weighted skin parameter for each body part. The second computing unit further calculates physiologically relevant parameters, including respiration rate and heart rate based on the computed time series of skin parameters. The display device also displays the live video feed of the baby.

The system may use an algorithm for ambient light correction. The algorithm is known to be relatively sensitive to subject motion, causing intense white lines along the edges of significantly moving objects. Therefore, by default, the algorithm will establish the absolute colour based on intervals of minimal motion. This method identifies which frames within a sequence have little motion artefacts and are therefore best suited for ambient light suppression. Since this algorithm establishes a figure of merit to the amount of motion between frames, it is relatively straightforward to establish whether the subject, such as a baby in the incubator is passive, active, or in distress based on the rate and frequency of motion. Therefore, combined reporting on colour and motion profiles may provide improve reporting and improved patient outcomes.

Another exemplary embodiment of the invention features an adaptive phototherapy treatment system comprising a skin Bilirubin monitoring module and a phototherapy treatment module. The skin Bilirubin monitoring module comprises a modulating light emitter unit, a camera unit, a computing unit and an interface unit for sending output signal to treatment module; and the phototherapy treatment module comprise, a light emitter unit, a control unit and an interface unit for receiving input signal from monitoring module. Here, the light emitter unit used (for L^{∗}a^{∗}b^{∗} colour assessment) contains spectral components that are capable of resolving the relevant skin discolorations caused by skin Bilirubin with sufficient specificity. Therefore, relatively broadband light emitter spectra are required.

For the assessment of the presence of bilirubin especially, the presence of sufficient blue light around 450 nm wavelength is relevant. For that purpose, the light emitter in this embodiment uses 'cold' white LEDs that provide sufficient blue and green spectral components needed to properly resolve the changes in yellow and red skin reflectance and that can be modulated without shifting the effective emission spectrum. In particular, LED sources with a light temperature in excess of 5000K can be suitably employed. Even though the colour rendering of these light emitter is far from 100%, using standard white balance calibration combined with the proposed ambient light correction technique is sufficient to establish a reasonably accurate L^{∗}a^{∗}b^{∗} quantification.

During skin Bilirubin measurement, the baby is illuminated briefly (or continuously) with modulated light emitter and the camera unit captures a few video frames of the baby. The video frames are processed such that the ambient light is removed and the frames are segmented into anatomical parts using an image recognition algorithm, as per the method described herein. Here an ROI, e.g. forehead, is defined, and yellowness is measured within the ROI. The system can then, for example by using a lookup table, relate yellowness to skin Bilirubin can be used to convert the measurement ROI yellowness to skin Bilirubin value.

By tracking the skin Bilirubin value, and based on a user-defined threshold value, the phototherapy treatment module can be adapted, e.g. by reducing or increasing intensity of, or disabling, light output.

Another exemplary embodiment of the invention features camera-based vital signs baby monitoring system comprising a modulating light emitter unit, a camera unit, a computing unit a user interface unit and a display unit. The spectral emission of the light emitter is configured to enhance detection of cutaneous blood.

During skin cutaneous blood measurement, the baby is illuminated briefly (or continuously) with modulated light emitter and the camera unit captures a few video frames of the baby. The video frames are processed such that the ambient light is removed and the image is segmented into anatomical parts using an image recognition algorithm. Here an ROI, e.g. cheek area, is defined, and PPG is measured within the ROI. Other parameters related to cutaneous blood could be measured, e.g. blood oxygenation. Also the detected motion of the baby could be used as relevant measurement parameter, e.g. respiration, stress-related motion, etc. These parameters may be displayed in the display unit.

The skilled person would be readily capable of developing a processor for carrying out any herein described method. Thus, each step of a flowchart may represent a different action performed by a processor, and may be performed by a respective module of the processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A computer implemented method (100) suitable for monitoring at least one skin region of a subject capable of moving relative to a source of ambient light, the method comprising:
receiving (102) a sequence of video frames (200) of the subject captured while a light emitter is controlled to provide a varying illumination to the subject;
generating (106), using a varying exposure between video frames of a first part (200A) of the sequence of video frames caused by said varying illumination during the capture of said first part, a first ambient light-corrected image (202A);
segmenting (108) the first ambient light-corrected image to generate a first segmented image (204A) in which the at least one skin region (206A, 208A, 210A) is identified;
generating (110), using a varying exposure between video frames of a second part (200B) of the sequence of video frames caused by said varying illumination during the capture of said second part, a second ambient light-corrected image (202B), the second part being temporally distinct from the first part;
segmenting (112) the second ambient light-corrected image to generate a second segmented image (204B) in which said at least one skin region (206B, 208B, 210B) is identified.

2. The computer implemented method (100) according to claim 1, further comprising:
extracting (114), from one or more of the first and second segmented images (204A, 204B), at least one skin parameter; and
outputting (116) the at least one skin parameter.

3. The computer implemented method (100) according to claim 2, wherein the skin parameter is a measure of one or more of: skin colour, skin texture, skin shape, and skin optical reflectance.

4. The computer implemented method (100) according to claim 2 or claim 3, comprising determining (124), using the sequence of video frames, an image quality metric, wherein the extracting (114) or the outputting (116) of said skin parameter is implemented responsive to the image quality metric meeting or exceeding a predetermined threshold.

5. The computer implemented method (100) according to claim 4, comprising issuing (126), responsive to the image quality metric failing to meet said predetermined threshold, a report indicating unreliable image data; or outputting (114), responsive to the image quality metric meeting/exceeding the predetermined threshold, said skin parameter relevant to the at least one skin region.

6. The computer implemented method (100) according to any of the preceding claims, wherein the first and second segmented images (204A, 204B) each comprise a first skin region (206A, 206B) and a second skin region (208A, 208B), the method comprising:
comparing (118) the first skin region (206A) identified from the first segmented image (204A) to the first skin region (206B) identified from the second segmented image (204B) to determine a first image consistency parameter;
comparing (120) the second skin region (208A) identified from the first segmented image to the second skin region (208B) identified from the second segmented image to determine a second image consistency parameter; and
comparing (122) the first and second image consistency parameters.

7. The computer implemented method (100) according to claim 6 when dependent on any of claims 2 to 5, comprising selecting the first skin region or the second skin region based on said comparison (122) of the first and second image consistency parameters, said outputted skin parameter being relevant to the selected skin region.

8. The computer implemented method (100) according to any of the preceding claims, further comprising:
obtaining RGB values of the first segmented image (204A) and/or the second segmented image (204B); and
converting the RGB values to CIE L^{∗}a^{∗}b^{∗} values.

9. The computer implemented method (100) according to any of the preceding claims, wherein the varying illumination is a pulsed illumination; optionally wherein a pulse frequency of the pulsed illumination is at a frequency of at least 70Hz.

10. The computer implemented method (100) according to any of the preceding claims, wherein a frame rate of the sequence of video frames is at least 24 frames per second.

11. The computer implemented method (100) according to any of the preceding claims, wherein the video frames are rolling shutter camera-captured video frames.

12. The computer implemented method (100) according to any of the preceding claims, wherein a timing of the varying illumination relative to a video frame capture timing is such that each of the video frames (200) comprises more exposed areas caused by the varying illumination and less exposed areas whose exposure is due only to the source of ambient light, the more exposed and the less exposed areas being positioned differently between the video frames of the first part (200A), and the more exposed and the less exposed areas being positioned differently between the video frames of the second part (200B),
wherein the first ambient light-corrected image (202A) is generated based on a comparison between the more exposed areas (212A) and the less exposed areas (214A) of the first part of the sequence of video frames, and
wherein the second ambient light-corrected image (202B) is generated based on a comparison between the more exposed areas (212B) and the less exposed areas (214B) of the second part of the sequence of video frames.

13. A computer program product comprising computer program code which, when executed on a computing device having a processing system, causes the processing system to perform all of the steps of the method (100) according to any of claims 1 to 12.

14. A system (300) for monitoring at least one skin region of a subject capable of moving relative to a source of ambient light, the system comprising:
a light emitter (302);
an image capture unit (304); and
a processing system (308) configured to:
receive a sequence of video frames of the subject captured by the image capture unit while the light emitter provides a varying illumination to the subject;
generate, using a varying exposure between video frames of a first part of the sequence of video frames caused by said varying illumination during the capture of said first part, a first ambient light-corrected image;
segment the first ambient light-corrected image to generate a first segmented image in which the at least one skin region is identified;
generate, using a varying exposure between video frames of a second part of the sequence of video frames caused by said varying illumination during the capture of said second part, a second ambient light-corrected image, the second part being temporally distinct from the first part; and
segment the second ambient light-corrected image to generate a second segmented image in which said at least one skin region is identified.

15. The system (300) according to claim 14, wherein the processing system (308) is further configured to control the image capture unit (304) to capture said sequence of video frames; and/or to control the light emitter (302) to provide said varying illumination to the subject.
